(19) Europäisches Patentamt
European Patent Office
Office européen des brevets

(11) EP 0 713 489 B1

(12) **EUROPEAN PATENT SPECIFICATION**

(45) Date of publication and mention of the grant of the patent:
**02.11.2000 Bulletin 2000/44**

(21) Application number: **94922335.8**

(22) Date of filing: **11.08.1994**

(51) Int. Cl.$^7$: **C07D 491/04**, C07D 493/04, C07D 495/04, G03C 1/72 // (C07D491/04, 311:00, 209:00), (C07D493/04, 311:00, 307:00), (C07D495/04, 311:00, 333:00)

(86) International application number:
**PCT/GB94/01758**

(87) International publication number:
**WO 95/05382 (23.02.1995 Gazette 1995/09)**

(54) **PHOTOCHROMIC HETEROCYCLOCHROMENES**
PHOTOCHROME HETEROCYCLOCHROMENE
HETEROCYCLOCHROMENES PHOTOCHROMIQUES

(84) Designated Contracting States:
**AT BE CH DE DK ES FR GB GR IE IT LI LU MC NL PT SE**

(30) Priority: **13.08.1993 GB 9316857**

(43) Date of publication of application:
**29.05.1996 Bulletin 1996/22**

(73) Proprietor: **PILKINGTON PLC**
**St. Helens, Merseyside WA10 3TT (GB)**

(72) Inventors:
- **RICKWOOD, Martin**
  **Clark's Summit Pennsylvania (US)**
- **HEPWORTH, John, David**
  **Preston Lancashire PR2 7ER (GB)**
- **GABBUTT, Christopher, David**
  **Blackburn Lancashire BB2 7NY (GB)**

(74) Representative:
**Pendlebury, Anthony et al**
**PAGE, WHITE & FARRER**
**54 Doughty Street**
**London WC1N 2LS (GB)**

(56) References cited:
**EP-A- 0 401 958** **EP-A- 0 562 915**
**WO-A-93/10112**

- **J. PHOTOCHEM. PHOTOBIOL. A vol. 49, no. 1-2 , 1989 pages 75 - 78 YA. N. MALKIN ET AL. 'Quantitative Study of the Photostability of Spiropyrans'**
- **IZV. AKAD. NAUK SSSR, SER. KHIM. (TRANSL.) vol. 39, no. 2 , 1990 pages 236 - 242 YA. N. MALKIN ET AL. 'Quantitative Study of the Photostability of Spiropyrans'**
- **IZV. AKAD. NAUK SSR, SER. KHIM. (TRANSL.) vol. 38, no. 11 , 1989 pages 2297 - 2302 T.B. KRASIEVA ET AL. 'Photochemistry of Spiropyrans of the Dithiolane Series with polycondensed Chromene Fragments'**
- **IZV. AKAD. NAUK SSSR, SER. KHIM. (TRANSL.) vol. 37, no. 7 , 1988 pages 1385 - 1387 S.M. ALDOSHIN ET AL. 'Effect of the Nature of the Heteroatoms in the Spiro Group on the Structure of Spiropyrans and an X-Ray Study of Spiropyran of the Dithiolane Series C17H16O2S2'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7 (HRS)/3.6

## Description

The present invention relates to certain novel photochromic heterobenzopyran compounds, and to articles and compositions containing them.

Photochromism is a well-known physical phenomenon which is observed with certain classes of chemical compounds. A detailed discussion of this phenomenon can be found in "Photochromism : Molecules and Systems", Studies in Organic Chemistry 40, Edited by H. Durr and H. Bouas-Laurent, Elsevier 1990.

Pyran derivatives as a class of compounds are known to be capable of exhibiting a photochromic effect. For example, U.S. Patent No. 5066818 describes a group of novel reversible photochromic naphthopyran compounds having at least one ortho-substituted phenyl group at the 3-position of the pyran ring.

The prior art pyran derived photochromic compounds - as illustrated, for example, in U.S. Patent 5066818 - in the activated state exhibit relatively simple absorbance profiles (e.g. Gaussian and Lorenzian curves) and therefore tend to exhibit relatively pure colours, provided that the photochromic material itself is not coloured. For many commercial applications, typically for use in sunglasses, a more neutral brown or grey colouration is required. In order to achieve a more neutral colouration using the prior art photochromic compounds it is necessary to mix together two or more photochromic compounds. This can present disadvantages, both in terms of cost and in terms of the ease with which the photochromic compounds can be incorporated into the polymeric host material into which they are to be introduced.

We have now found a group of heterobenzopyran compounds which when dispersed into a solution or a polymeric host material and activated with actinic light exhibit a neutral, typically brown, colouration in the activated state.

According to the present invention there is provided a heterobenzopyran compound of general formula (I)

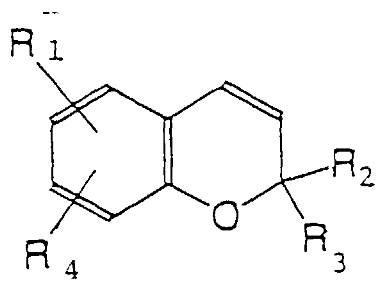

(I)

wherein $R_1$ represents a five-membered heterocyclic group containing one or more hetero atoms and fused either to the f-face or the g-face of the benzo moeity of the benzopyran skeleton, the said heterocyclic group being fused in such a manner that the hetero atom in the heterocyclic group is linked directly to a carbon atom on the said benzo moeity, and, optionally, the said heterocyclic group has fused thereto a further carbocyclic or heterocyclic group or is substituted with a group of formula $R_4$ as defined below;

each of the $R_2$ and $R_3$ substituents is chosen from a phenyl group, a 4-trifluoromethyl-phenyl group, a 4-alkoxyphenyl group, preferably 4-methoxyphenyl, a 2,4-di(alkoxy)phenyl group, preferably 2,4-dimethoxyphenyl, or a 4-dialkylamino-phenyl group, preferably 4-dimethylamino-phenyl, with the proviso that at least one of the $R_2$ and $R_3$ substituents is chosen from a 4-alkoxyphenyl group, preferably 4-methoxyphenyl, or a 2,4-di(alkoxy)phenyl group, preferably, 2,4-dimethoxyphenyl; and

$R_4$ represents a hydrogen atom or alkyl.

For the avoidance of doubt, throughout this specification, the ring numbering used to describe the benzopyran compounds of the present invention is as follows:-

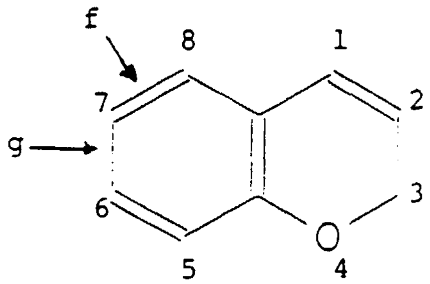

(II)

and the f-face on the benzo moeity of the benzopyran skeleton is the face between carbon atoms 7 and 8, and the g-face is that between carbon atoms 6 and 7 of the said benzo moeity.

Throughout this specification, unless stated otherwise, the term "alkyl" is to be taken to mean an alkyl group having from 1 to 6 carbon atoms. Similarly, the term "alkoxy" is to be taken to mean an alkoxy group having from 1 to 6 carbon atoms.

The heterobenzopyran compounds of the present invention possess extremely complex absorbance profiles (i.e. they have multiple absorbance peaks/shoulders/inflections) and as a consequence they are found to exhibit neutral colours such as browns when incorporated in solutions and polymers.

Some of the compounds in accordance with the invention are obtained as mixtures of isomers which are not readily separable although in other cases one isomer tends to predominate and can be separated out. In both cases, however, we have found that one obtains the complex absorbance profile mentioned above, so that both the isomeric mixtures and the single isomers exhibit the said neutral, typically brown, colouration.

Preferably, the five membered hererocyclic ring in the group $R_1$ is a furan ring, a pyrrole ring or a thiophene ring, preferably with the hetero-atom attached to the 7-position of the benzopyran skeleton.

Preferred substituents on the heterocyclic ring in the group $R_1$ are a benzene ring fused thereto, or a cyclohexane ring fused thereto, or one or more alkyl substituents on the heterocyclic ring.

In the case of the pyrrole ring, the ring nitrogen atom may carry an alkyl substituent, usually a methyl group.

Preferred $R_4$ groups are hydrogen or alkyl, typically methyl.

The benzopyran compounds of the present invention may be prepared by a general preparative method which is based on the following reaction scheme:-

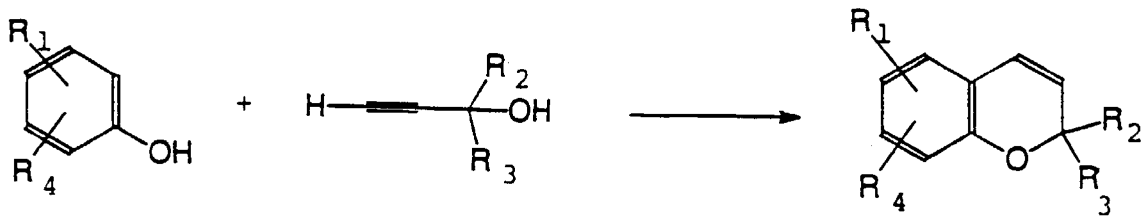

(III) (IV) (I)

The preparative methods used to form the hetero benzopyran compounds of the present invention are closely analogous to the methods used to prepare relatively well known photochromic benzo- and naphthopyran compounds. The synthesis of such known benzopyran and naphthopyran compounds is described in detail, for example, by L. Merlini in Advances in Heterocyclic Chemistry, 1975, 18, 159, and in a number of patents, for example, U.S. 5066818, U.S. 4990287, U.S. 4980089 and WO 92/09593. Typically, the heterobenzopyrans of the present invention are formed by reacting a hydroxy compound of general formula (III) with a propargyl alcohol of general formula (IV) in the presence of acidic alumina (e.g. Brockmann 1 alumina), trifluoroacetic acid or other like acidic catalyst; the initial condensation/etherification reaction between the hydroxy compound (III) and the propargyl alcohol of formula (IV) is followed by a series of catalysed sigmatropic shifts and tautomerisation to yield the desired compound of general formula (I).

The synthetic process for preparing the heterobenzopyran compounds of the present invention can lead to the formation of two isomeric heterobenzopyrans of formulae (IA) and (IA) as illustrated in the following reaction scheme:-

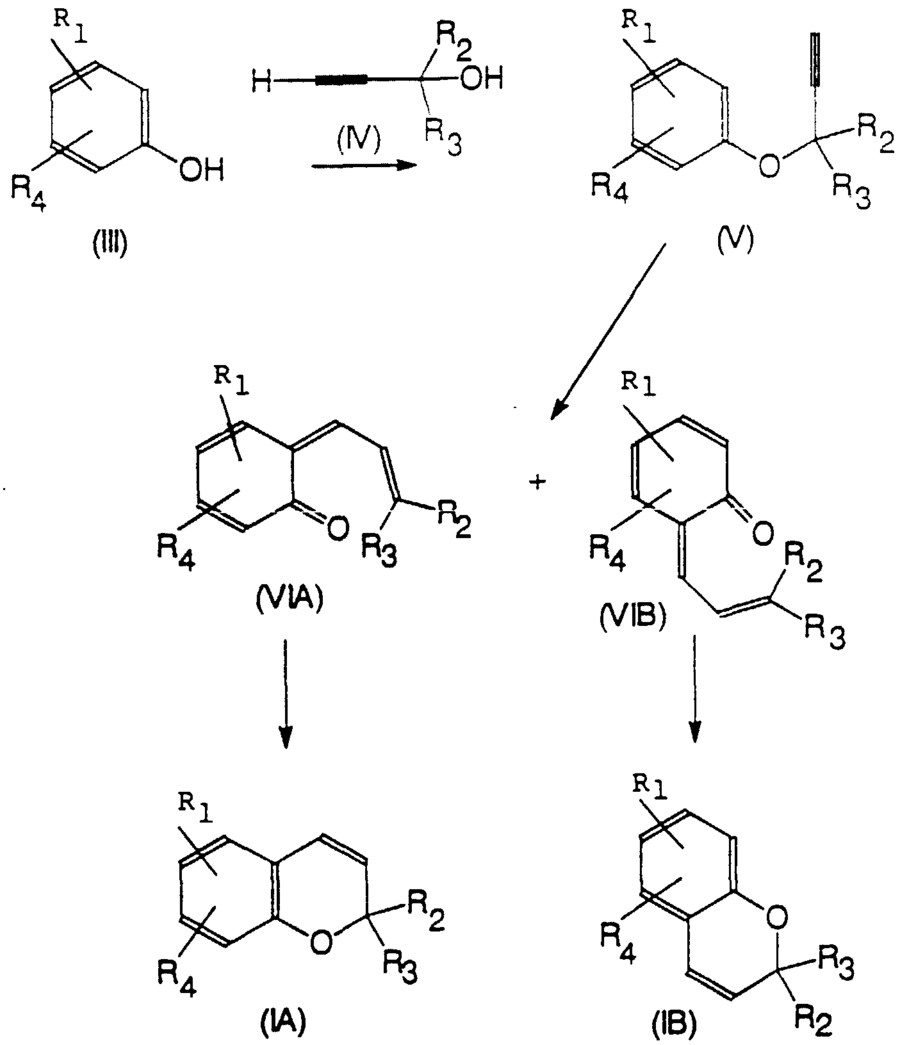

The reaction of the hydroxy compound (III) with the propargyl alcohol (IV) proceeds via the initial formation of an aryl propargyl ether of formula (V), followed by a Claisen-like [3,3]- sigmatropic re-arrangement. When there are no substituents in the ortho positions on either side of the hydroxy group in the compound of formula (III), the Claisen-like [3,3]-sigmatropic re-arrangement gives rise to two isomeric intermediates of formulae (VIA) and (VIB), and these intermediates then undergo a [1,5]- sigmatropic shift to form the two isomeric heterobenzopyran compounds of formulae (IA) and (IB).

The presence of substituents or fused moieties on the benzene ring of the compound of formula (III) can lead to a predominance of one of the two isomers (IA) and (IB) since the isomeric ratio of the two heterobenzopyran isomers is affected by the regioselectivity of the cyclisation of the aryl propargyl ether of formula (V).

The hydroxy compounds of formula (III) are either commercially available compounds or are prepared using known synthetic methods, or methods derived from these known synthetic methods.

For example, 5-hydroxy-2,3-dimethylbenzothiophene, a starting material in Example 8 below, is made by a method analogous to that described by S. Gronowitz et al. (Acta. Pharm. Suec. 1978, 15, 337) for the preparation of 2-hydroxydibenzothiophene. Thus, 5- bromo-2,3-dimethylbenzo thiophene is converted using n-BuLi to the 5-Lithium compound which is reacted with $(n\text{-}BuO)_3B$ to form the corresponding tri-butyl borate derivative, which is then treated with $H_20_2$ to yield the desired 5-hydroxy-2,3-dimethyl thiophene.

The propargyl alcohols of general formula (IV) which are used to prepare the heterobenzopyran compounds

of the present invention are prepared in known manner, e.g. as described by T.F. Rutledge in Acetylenic Compounds, Reinhold, New York 1968.

The novel heterobenzopyran compounds of the present invention are found to be particularly useful as photochromic materials to be incorporated into polymeric host materials so as to impart photochromic properties to the said polymeric host materials.

The photochromic heterobenzopyran compounds of the present invention are incorporated into the plastics host material in known manner, for example as described in European Patent No. 0245020 or U.S. Patent No. 5066818.

The materials of the present invention impart a yellow-brown, red-brown or olive-green colouration in their darkened state. In the faded or bleached condition the materials exhibit a colourless or pale colouration.

Typical polymeric host materials are optically clear polymer materials, such as polymers of polyol(allyl carbonate)-monomers, polyacrylates such as polymethylmethacrylates, cellulose acetate, cellulose triacetate, cellulose acetate propionate, cellulose acetate butyrate, poly(vinyl acetate), poly(vinyl alcohol), polyurethanes, polycarbonates, polyethylene terephthalate, polystyrene, styrene/methylmethacrylate copolymers, styrene/acrylonitrile copolymers, and polyvinylbutyral. Transparent copolymers and blends of the transparent polymers are also suitable as host materials. Polymers of the type described in EP 0294056 or EP 0453149 are also suitable.

Preferably, the polymeric host material is an optically clear polymerized organic material such as a polyurethane or a polymer of diethylene glycol bis(allyl carbonate) (sold under the trade name CR-39), or SPECTRALITE - a material sold by Sola Optical USA.

Usually, the amount of photochromic benzopyran compound incorporated in the polymeric host material ranges from 0.01 to 0.5 wt%, based on the weight of the polymeric host material.

In some applications, it may be desirable or advantageous to combine the heterobenzopyran compounds of the present invention with other photochromic materials to obtain an aggregate colour effect. For example, spiro-oxazine materials may have a colour range of 530 to 680 nm which means that in the darkened condition the spiro-oxazines impart a red-purple or purple or blue or blue-green or green colouration to a host material. Thus, the present heterobenzopyran compounds can be combined with known spiro-oxazine materials such as those described in our European Patent No. 0245020, or in our UK Patent Applications Nos. 92/25346, 92/25347 and 92/25348, or with the spiro (indolino) naphthoxazines, spiro (indolino) pyrido benzoxazines and spiro (indolino) benzoxazines described in U.S. Patents Nos. 4637698, 3562172, 3578602, 4816584, 4215010 and 4342668 in order to modify the colouration in the darkened condition from a brown to a grey. For similar reasons, the compounds of the present invention may also be combined with other photochromic materials such as the naphthopyran compounds described in our UK Patent Application No. 9306587 or in U.S. Patent No. 5066818.

Typically, when used in combination, the further additional photochromic material is present in an amount of from 0.001 to 0.5 weight %, based on the weight of the polymeric host material.

Examples of suitable uses of the photochromic plastic articles incorporating the heterobenzopyran compounds of the invention are in the manufacture of piano lenses, e.g. for sunglasses, and ophthalmic lenses and as photochromic transparencies for vehicles such as cars and aircraft.

The following Examples illustrate the present invention. The structures of the various products were determined using proton NMR.

Example 1

3,3-Dianisyl-3H-[chromeno[6,5-b]benzofuran] (1a) and 3,3-dianisyl-3H-[chromeno[6,7-b]benzofuran] (1b).

A mixture of 2-hydroxydibenzofuran (2.38g; 0.0129mol), 1,1-dianisylprop-2-yn-1-ol (3.35g; 0.0129mol) and acidic Alumina Brockmann I (8.0g) in benzene (150ml) was heated under reflux for 1.5h. The resulting dark mixture was filtered to remove the alumina which was washed with benzene, the combined filtrate and washings were evaporated and the residue flash chromatographed over silica (eluent: 20% ethyl acetate in hexane) to give a pale gum which upon trituration with pet. ether (60/80)/diethyl ether afforded a 2:1 isomeric mixture of 3,3-dianisyl-3H-[chromeno[6,5-b]benzofuran of formula (1a) and 3,3-dianisyl-3H-[chromeno[6,7-b]benzofuran of formula (1b) as a white solid (1.25g; 22%; m.pt. 120-49°C). Fractional recrystallisation of the mixture with pet.ether (60-80)/ethyl acetate afforded pure 3,3-dianisyl-3H-[chromeno[6,5-b] benzofuran] (1a) as a white solid (0.65g; m.pt. 159-60°C).

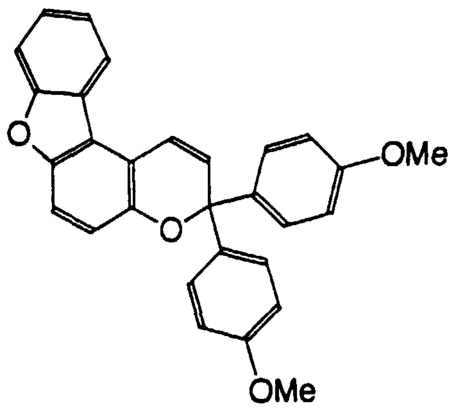

(1a)

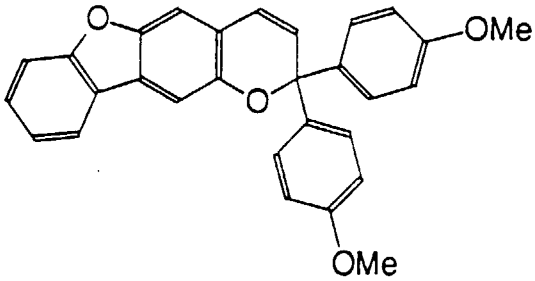

(1b)

Example 2

3-Anisyl-3-(2,4-dimethoxyphenyl)-3H-[chromeno[6,5-b]benzofuran] (2a) and 3-anisyl-2-(2,4-dimethoxyphenyl)-3H-[chromeno[6,7-b] benzofuran] (2b)

A mixture of 2-hydroxydibenzofuran (0.62g; 0.0036mol), 1-anisyl-1-(2,4- dimethoxyphenyl)prop-2-yn-1-ol (1.0g; 0.0036mol) and acidic Alumina Brockmann I(5.0g) in benzene (40ml) was heated under reflux for 1.5h. The resulting dark mixture was filtered to remove the alumina which was washed with benzene, the combined filtrate and washings were evaporated and the residue flash chromatographed over silica (eluent: 20% ethyl acetate in hexane) to give a 2:1 isomeric mixture of 3-anisyl-3-(2,4- dimethoxyphenyl)-3H-[chromeno [6,5-b]benzofuran] of formula (2a) and 3-anisyl-3-(2,4-dimethoxyphenyl)-3H-[chromeno [6,7-b]benzofuran] of formula (2b) as a pale yellow glass of m.pt. 78-83°C. The isomers could not be separated.

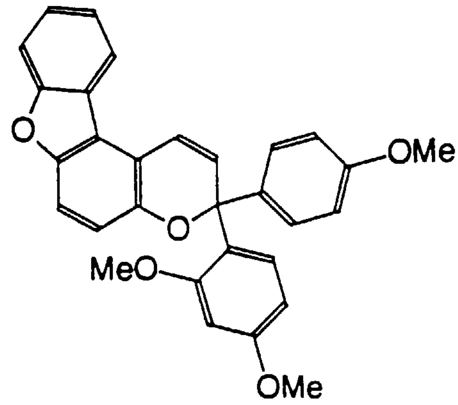

(2a)

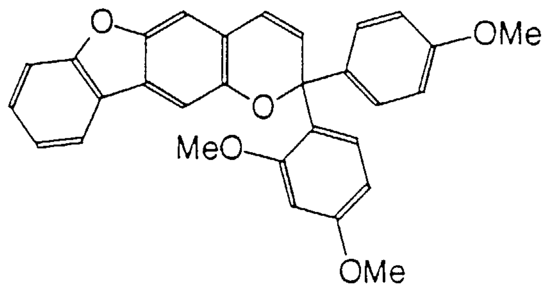

(2b)

Examples 3 and 4

Using preparative procedures analogous to those used in Examples 1 and 2, the following benzopyrans in accordance with the invention were also prepared:-

Example 3

3,3-Dianisyl-6-methyl-3H-[pyrano[2,3-b]-9H-5,6,7,8-tetrahydrocarbazole] (3a) and 3,3-dianisyl-10-methyl-3H-[pyrano[3,2-b] -9H-5,6,7,8-tetrahydrocarbazole] (3b)

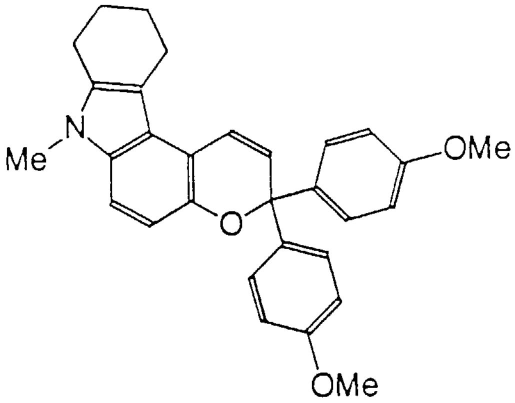

(3a)

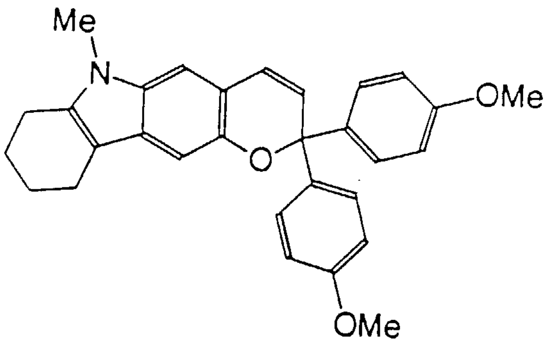

(3b)

The structures of these compounds were elucidated using n.m.r. spectra. The melting point of compound (3a) was determined as 147-155°C and that of compound (3b) was 205-207°C.

Example 4

3,3-Dianisyl-7H-[pyrrolo[2,3-f]chromenel (4)

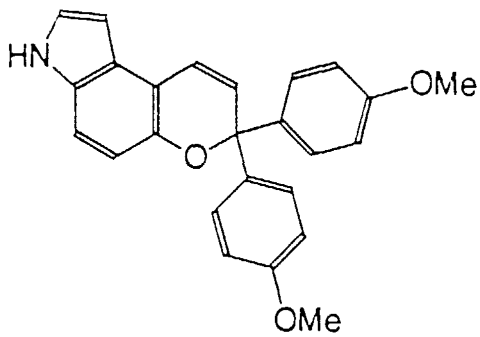

( 4)

The structure of this compound was determined from its n.m.r. spectrum. It exhibited a melting point of 145-152°C (decomp).

Example 5

3-Anisyl-3-(2,4-dimethoxyphenyl)-6-methyl 3H-[chromeno[6,7-b] benzofuran] (5)

A solution of 2-hydroxydibenzofuran (18.42g;0.10mol) and 25-30% aqueous dimethylamine (22.0ml;0.122mol) in ethanol (100ml) was treated dropwise over 1h with 37% aqueous formaldehyde (9.0ml;0.1mol). The mixture was then heated at 100°C for 1h. The mixture was cooled to room temperature to precipitate 1-dimethylaminomethyl-2- hydroxydibenzofuran (5a). The product was washed firstly with 50% aqueous ethanol and then with ice-cold ethanol to give the product as tan coloured crystals (18.6g;77%), m.pt 113-5°C.

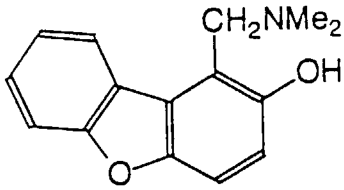

(5a)

A suspension of 1-dimethylaminomethyl-2-hydroxydibenzofuran (5a) (5.0g;0.021mol) in 10% aqueous potassium hydroxide (50ml) at 60°C (bath temp 100°C) was treated with portions of Nickel-Aluminium alloy (50:50) powder over 0.5h at such a rate as to maintain boiling. The mixture was heated at 100°C for a further 1h, then cooled to room temperature and filtered through a pad of Celite and the filter cake washed with water. Acidification of the filtrate with conc. HCl gave a precipitate. The aqueous suspension was extracted with ethyl acetate, the extracts were combined, dried and evaporated to afford the crude product (3.2g). Purification using flash-chromatography over silica (eluent: 10% ethyl acetate in petroleum ether 60/80) gave 1-methyl-2-hydroxydibenzofuran (5b) (TLC; on silica; eluent 10% ethyl acetate in petroleum ether 60/80; $R_f$ = 0.2) as an off-white solid, m.pt. 135.5-6.7°C.

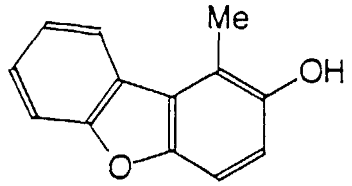

(5b)

A mixture of 1-methyl-2-hydroxydibenzofuran (5b) (0.60g; 0.0030mol), 1-anisyl-1-(2,4-dimethoxyphenyl)prop-2-yn-1-ol (0.90; 0.0030mol) and acidic Alumina Brockmann I (3.3g) in benzene (40ml) was heated under reflux for 15min. The resulting dark mixture was filtered to remove the alumina which was washed with benzene, the combined filtrate and washings were evaporated and the residue flash chromatographed over silica (eluent:20% ethyl acetate in hexane) to give a pale gum which upon trituration with hexane gave 3-anisyl-3-(2,4-dimethoxyphenyl)-6-methyl 3H-[chromeno [6,7-b]benzofuran] (5) as a crude product (0.42g;29%; m.pt. 158-65°C). Further purification by recrystallisation (hexane/ethyl acetate) gave the product as colourless microcrystals, m.pt. 173-4.7°C.

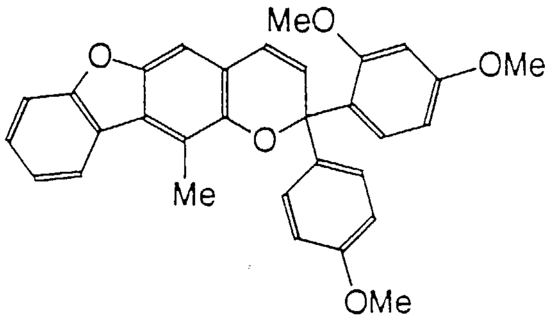

(5)

Example 6

3-Anisyl-3-(2,4-dimethoxyphenyl)-8,9-dimethyl-3H-[pyrano[3,2-e] benzothiophene] (6)

A solution of 5-bromo -2,3-dimethylbenzo[b]thiophene (3,61g; 0.015mol) in diethyl ether (40ml) under nitrogen was cooled to 0°C and treated dropwise over 5 min. with 2.5M n-butyl lithium (6.1ml;0.0153mol). The resulting mixture was stirred for a further 20 min. then treated over 10 min. with a diethyl ether solution of tributyl borate (3.45g; 4.1ml; 0.0153mol) and allowed to warm to room temperature and stirred for 110min. The mixture was cooled to 10°C and treated with acetic acid (1.3ml;0.0225mol) in one portion. After further cooling to 0°C, the mixture was treated dropwise with a solution of 30% hydrogen peroxide (1.7ml, 0.0165mol) in water (10ml) which resulted in an exotherm (T=15°C). The mixture was allowed to warm to room temperature and stirred for 2 hours. The mixture was poured into a saturated aqueous solution of ammonium sulphate containing ferrous ammonium sulphate and the organic phase washed until no colour was present. The organic phase was dried and evaporated to leave a tacky solid (contaminated with n-butanol) which upon trituration with petroleum ether (30/40) gave 5-hydroxy -2,3-dimethylbenzo[b]thiophene (6a). [TLC 20% ethyl acetate in hexane over silica; $R_f$ 0.22] as a white solid (1.42g;53%).

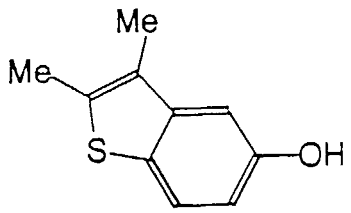

(6a)

A mixture of 5-hydroxybenzothiophene (6a) (0.40g; 0.00224mol), 1-anisyl-1-(2,4-dimethoxyphenyl)prop-2-yn-1-ol (0.66g; 0.00224mol) and acidic Alumina Brockmann I (3.50g) in toluene (40ml) was heated under reflux for 1h. The resulting dark mixture was filtered to remove the alumina which was washed with toluene, the combined filtrate and washings were evaporated and the residue flash chromatographed over silica (eluent: 20% ethyl acetate in hexane) to give a yellow oil which crystallised upon trituration with hexane to afford 3-anisyl-3-(2,4-dimethoxyphenyl)-8,9-dimethyl-3H-[pyrano[3,2-e] benzothiophene] (6) as a white solid (0.32g;31%) m.pt. 135-37°C.

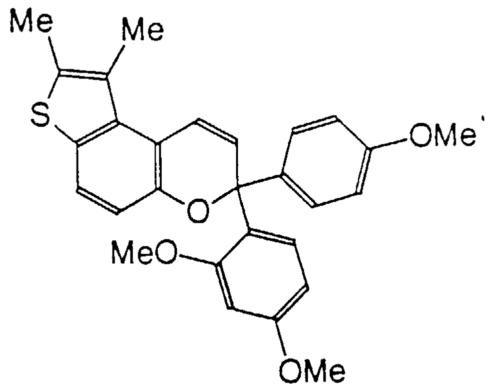

(6)

The photochromic properties of the heterobenzopyran compounds of the present invention were tested by preparing, in conventional manner, by a direct casting process, 1.0 mm plates of a polyurethane host material of the type described in EP 0294056, incorporating the photochromic heterobenzopyran in a concentration of 0.3% w/w.

The resultant plates were illuminated under standard solar simulation conditions at Air Mass 2 at 2l°C (see Parry Moon, J. Franklin Inst. 230, (1940), p 583-617). The measurements which were made on the samples in the darkened condition were taken when the samples had reached a steady state; this steady state was deemed to have been reached after 10 minutes in the darkened condition.

The results obtained are shown in Table I.

Table 1 shows the difference between the unactivated and the activated photochromic article.

The first data column shows the induced optical density (IOD), measured at the integrated visible transmission (IVT), which is the log difference between the bleached transmission (BT) and the darkened transmission (DT).

$$IOD\ (IVT) = \log BT\ (\%)/DT(\%)$$

The two columns to the right of the table give both a subjective description of the bleached and darkened states and an objective description of colour using the universal Lab system (see reference at foot of Table 1). The Lab system is a method for representing colours mathematically and therefore numerically.

As will be seen from the results given in Table 1, the colouration of the samples in the darkened condition is a brown, yellow-brown, orange-brown or olive-green colouration.

Table 1

| 0.3 % w/w of photochromic in polyurethane plate (1mm) | | | | | |
|---|---|---|---|---|---|
| Example | Induced Optical Density[1] *(Bleached Transmission/Darkened transmission)* | Bleached colour[2] | | Darkened colour[2] | |
| | (IVT) | a* | b* | a* | b* |
| 2 | 0.730 | colourless | | brown | |
| | *(89.5/16.7)* | -0.87 | 0.70 | 16.81 | 18.33 |
| 3b | 0.286 | colourless | | yellow brown | |
| | *(88.9/46.0)* | -0.99 | 2.59 | 15.43 | 60.42 |
| 4 | 0.650 | pale brown | | warm brown | |
| | *(75.5/16.9)* | -1.31 | 12.46 | 26.24 | 49.14 |
| 5 | 0.718 | colourless | | olive green | |
| | *(91.1/17.4)* | -1.09 | 0.91 | -7.10 | 32.62 |
| 6 | 0.346 | colourless | | yellow brown | |
| | *(89.5/40.4)* | -0.83 | 0.92 | 21.21 | 51.42 |

[1] 10 minutes darkening under Xenon arc filtered to Air-Mass 2 at 21°C.
[2] L*a*b* system (DIN 6174, Commission Internationale de L'Eclairage 1976) developed by Judd and Hunter (ref. G.J. Chamberlin and D.C. Chamberlin, Colour: Its Meassurement, Computa tion and Application, ed. L.C. Thomas, Heyden 1980, ch. 4.

## Claims

**1.** A heterobenzopyran compound of general formula (I)

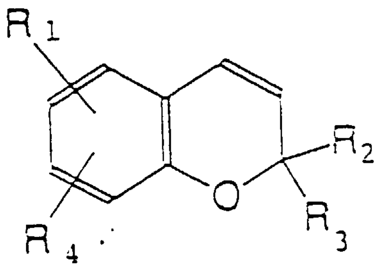

(I)

wherein $R_1$ represents a five-membered heterocyclic group containing one or more hetero atoms and fused either to the f-face or the g-face of the benzo moiety of the benzopyran skeleton, the said heterocyclic group being fused in such a manner that the hetero atom in the heterocyclic group is linked directly to a carbon atom on the said benzo moiety, and, optionally, the said heterocyclic group has fused thereto a further carbocyclic or heterocyclic group or is substituted with a group of formula $R_4$ as defined below;

each of the $R_2$ and $R_3$ substituents is chosen from a phenyl group, a 4-trifluoromethyl-phenyl group, a 4-alkoxyphenyl group, a 2,4-di(alkoxy)phenyl group or a 4-dialkylamino-phenyl group, with the proviso that at least one of the $R_2$ and $R_3$ substituents is chosen from a 4-alkoxyphenyl group or a 2,4-di(alkoxy)phenyl group; and

$R_4$ represents a hydrogen atom or alkyl.

2. A heterobenzopyran compound according to claim 1 wherein the $R_2$ and $R_3$ substituents are chosen from a 4-methoxyphenyl group, a 2,4-dimethoxyphenyl group and a 4-dimethylaminophenyl group with the proviso that at least one of the $R_2$ and $R_3$ substituents is chosen from a 4-methoxyphenyl group or a 2,4-dimethoxy phenyl group.

3. A heterobenzopyran compound according to claim 1 or claim 2, wherein the five membered heterocyclic ring in the group $R_1$ is a furan ring, a pyrrole ring or a thiophene ring.

4. A heterobenzopyran compound according to claim 3 wherein the heteroatom of the furan, pyrrole or thiophene ring is attached to the 7-position of the benzopyran skeleton.

5. A heterobenzopyran compound according to any preceding claim wherein the heterocyclic ring in the group $R_1$ has a benzene ring fused thereto, or a cyclohexane ring fused thereto, or carries one or more alkyl substituents.

6. A heterobenzopyran compound according to any one of claims 3 to 5 wherein the ring nitrogen atom of the pyrrole ring is substituted with an alkyl group.

7. A heterobenzopyran compound according to claim 6 wherein the alkyl group is a methyl group.

8. A heterobenzopyran compound according to any one of the preceding claims, wherein $R_4$ is a hydrogen atom or a methyl group.

9. 3,3-Dianisyl-3H-[chromeno[6,5-b]benzofuran].

10. 3,3-Dianisyl-3H[chromeno[6,7-b]benzofuran].

11. 3-Anisyl-3-(2,4-dimethoxyphenyl)-3H-[chromeno[6,5-b] benzofuran].

12. 3-Anisyl-3-(2,4-dimethoxyphenyl)-3H-[chromeno[6,7-b] benzofuran].

13. 3,3-Dianisyl-6-methyl-3H-[pyrano[2,3-b]-9H-5,6,7,8-tetrahydrocarbazole].

14. 3,3-Dianisyl-10-methyl-3H-[pyrano[3,2-b]-9H-5,6,7,8-tetrahydrocarbazole].

15. 3,3-Dianisyl-7H[pyrrolo[2,3-f]chromene].

16. 3-Anisyl-3-(2,4-dimethoxyphenyl)-6-methyl-3H-[chromeno[6,7-b]benzofuran].

17. 3-Anisyl-3-(2,4-dimethoxyphenyl)-8,9-dimethyl-3H-[pyrano [3,2-e]benzothiophene].

18. A process for preparing a heterobenzopyran compound of formula (I) as defined in claim 1, which process comprises condensing a hydroxy compound of general formula (III)

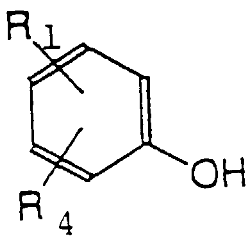

(III)

wherein $R_1$ and $R_4$ are as defined in claim 1, with the hetero atom in the heterocyclic ring of the group $R_1$ being located in the para- or meta- position relative to the hydroxy group on the benzene ring, with a propargyl alcohol of general formula (IV)

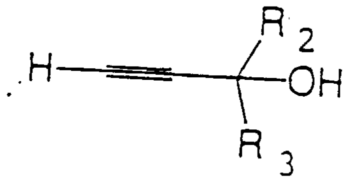

(IV)

wherein $R_2$ and $R_3$ are as defined in claim 1, in the presence of acidic alumina, trifluoroacetic acid or another like acidic catalyst.

**19.** A photochromic article comprising a polymeric host material having a heteropyran compound as defined in any one of claims 1 to 17 incorporated therein or applied thereto.

**20.** A photochromic article according to claim 19, wherein the polymeric host material is selected from polymers of polyol (allyl carbonate) monomers, polyacrylates, poly(alkylacrylates), cellulose acetate, cellulose triacetate, cellulose acetate propionate, cellulose acetate butyrate, poly(vinyl acetate), poly(vinyl alcohol), polyurethanes, polycarbonates, polyethylene terephthalate, polystyrene, styrene/methylmethacrylate copolymers, styrene/ acrylonitrile copolymers, and polyvinylbutyral.

**21.** A photochromic article according to claim 20, wherein the polymeric host material is a polyurethane or a polymer of diethyleneglycol bis (allyl carbonate).

**22.** A photochromic article according to claim 19, 20 or 21, wherein the amount of heterobenzopyran compound is from 0.01 to 0.5% by weight, based on the weight of the polymeric host material.

**23.** A photochromic article according to any one of claims 19 to 22, comprising a further photochromic compound selected from spiro(indoline)naphthoxazines, spiro(indolino)pyrido benzoxazines, spiro(indolino)benzoxazines, and naphthopyrans.

**24.** A photochromic article according to claim 23, wherein the further photochromic compound is present in an amount of from 0.001 to 0.5% by weight, based on the weight of the polymeric host material.

**25.** A photochromic article according to any one of claims 19 to 24, which is in the form of a lens.

**26.** A photochromic article according to claim 25, wherein the lens is an ophthalmic lens.

**Patentansprüche**

**1.** Heterobenzopyran-Verbindung der allgemeinen Formel (I),

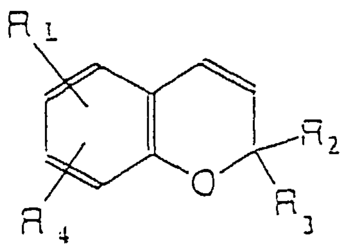

(I)

bei der $R_1$ eine heterozyklische, fünfgliedrige Gruppe darstellt, die ein oder mehrere Heteroatome enthält und entweder über die f-Seite oder g-Seite der Benzo-Einheit des Benzopyran-Gerüsts verknüpft ist, wobei die heterozyklische Gruppe so verknüpft ist, dass das Heteroatom der heterozyklischen Gruppe direkt mit dem

Kohlenstoffatom an der entsprechenden Benzo-Einheit verbunden ist und die betreffende heterozyklische Gruppe wahlweise mit einer weiteren carbozyklischen oder heterozyklischen Gruppe verknüpft ist oder mit einer Gruppe der Formel $R_4$ entsprechend der nachfolgenden Definition substituiert ist;

die Substituenten $R_2$ und $R_3$ sind jeweils ausgewählt aus einer Phenyl-Gruppe, einer 4-Trifluoromethylphenyl-Gruppe, einer 4-Alkoxyphenyl-Gruppe, einer 2,4-Dialkoxyphenyl-Gruppe oder einer 4-Dialkylaminophenyl-Gruppe unter dem Vorbehalt, dass mindestens einer der Substituenten $R_2$ und $R_3$ ausgewählt ist aus einer 4-Alkoxyphenyl-Gruppe oder einer 2,4-Dialkylamino-phenyl-Gruppe oder einer 2,4-Dialkoxyphenyl-Gruppe; und

$R_4$ ein Wasserstoffatom oder eine Methyl-Gruppe darstellt.

**2.** Heterobenzopyran-Verbindung gemäß Anspruch 1, bei der die Substituenten $R_2$ und $R_3$ ausgewählt sind aus einer 4-Methoxyphenyl-Gruppe, einer 2,4-Dimethoxyphenyl-Gruppe und einer 4-Dimethylaminophenyl-Gruppe unter dem Vorbehalt, dass mindestens einer der Substituenten $R_2$ und $R_3$ ausgewählt ist aus einer 4-Methoxyphenyf-Gruppe oder einer 2,4-Dimethoxyphenyl-Gruppe.

**3.** Heterobenzopyran-Verbindung gemäß Anspruch 1 oder Anspruch 2, bei der der fünfgliedrige, heterozyklische Ring in der Gruppe $R_1$ ein Furan-Ring, ein Pyrrol-Ring oder ein Thiophen-Ring ist.

**4.** Heterobenzopyran-Verbindung gemäß Anspruch 3, bei der das Heteroatom des Furan-, Pyrrol- oder Thipophen-Ringes mit der Position 7 des Benzopyran-Gerüstes verbunden ist.

**5.** Heterobenzopyran-Verbindung gemäß einem oder mehreren der vorangegangenen Ansprüche, bei der der heterozyklische Ring in der Gruppe $R_1$ mit einem Benzol-Ring oder einem Cyclohexan-Ring verknüpft ist oder einen oder mehrere Alkyl-Substituenten aufweist.

**6.** Heterobenzopyran-Verbindung gemäß einem oder mehreren der Ansprüche 3 bis 5, bei der das zum Ring gehörende Stickstoffatom des Pyrrol-Ringes mit einer Alkyl-Gruppe substituiert ist.

**7.** Heterobenzopyran-Verbindung gemäß Anspruch 6, bei der die Alkyl-Gruppe eine Methyl-Gruppe ist.

**8.** Heterobenzopyran-Verbindung gemäß einem oder mehreren der vorangegangenen Ansprüche, bei der $R_4$ ein Wasserstoffatom oder eine Methyl-Gruppe ist.

**9.** 3,3-Dianisyl-3H- [chromeno [6,5-b] benzofuran].

**10.** 3,3-Dianisyl-3H- [chromeno [6,7-b] benzofuran].

**11.** 3-Anisyl-3- (2,4-dimethoxyphenyl) -3H-[chromeno [6,5-b] benzofuran].

**12.** 3-Anisyl-3- (2,4-dimethoxyphenyl) -3H-[chromeno [6,7-b] benzofuran].

**13.** 3,3-Dianisyl-6-methyl-3H- [pyrano [2,3-b] -9H-5,6,7,8 -tetrahydrocarbazol].

**14.** 3,3-Dianisyl-10-methyl-3H- [pyrano [3,2-b] -9H-5,6,7,8 -tetrahydrocarbazol].

**15.** 3,3-Dianisyl-7H- [pyrrolo [2,3-f] chromen].

**16.** 3-Anisyl - 3 - (2,4-dimethoxyphenyl) - 6 - methyl -3H- [chromeno [6,7-b] benzofuran].

**17.** 3-Anisyl - 3 - (2,4-dimethoxyphenyl) - 8,9 - dimethyl -3H- [pyrano [3,2-e] benzothiophen].

**18.** Verfahren zur Herstellung einer Heterobenzopyran-Verbindung der Formel (I) gemäß der Definition in Anspruch 1, wobei das Verfahren die Kondensation einer Hydroxy-Verbindung der allgemeinen Formel (III),

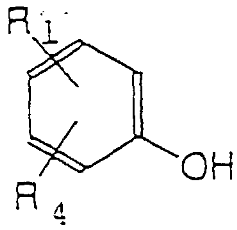

(III)

bei der $R_1$ und $R_4$ gemäß der Definition in Anspruch 1 vorliegen, mit dem Heteroatom, das in dem heterozyklischen Ring der Gruppe $R_1$ in para- oder meta-Position, bezogen auf die Hydroxy-Gruppe des Benzol-Ringes, vorliegt, in Gegenwart von essigsaurer Tonerde, Trifluoressigsäure oder einem ähnlich sauren Katalysator mit einem Propargyl-Alkohol der allgemeinen Formel (IV),

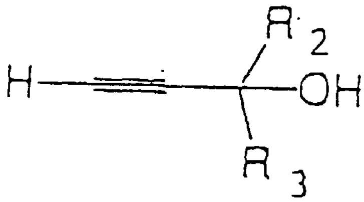

(IV)

in dem $R_2$ und $R_3$ gemäß der Definition in Anspruch 1 vorliegen.

**19.** Ein photochromer Gegenstand, der ein polymeres Trägermaterial umfasst, das eine heterozyklische Verbindung gemäß der Definition in einem oder mehreren der Ansprüche 1 bis 17 eingebaut oder angelagert enthält.

**20.** Ein photochromer Gegenstand gemäß Anspruch 19, bei dem das polymere Trägermaterial ausgewählt ist aus den Polymeren: Polyol(allylcarbonat)Monomere, Polyacrylate, Polyalkylacrylate, Celluloseacetat, Cellulosetriacetat, Celluloseacetat-proprionat, Celluloseacetat-butyrat, Polyvinylacetat, Polyvinylalkohol, Polyurethane, Polycarbonate, Polyäthylen, Terephthalat, Polystyren, Styren/Methylmethacrylat-Kopolymere, Styren/Acrylnitril-Kopolymere und Polyvinylbutyral.

**21.** Ein photochromer Gegenstand gemäß Anspruch 20, bei dem das polymere Trägermaterial ein Polyurethan oder ein Polymer des Diäthylenglycol-bis-allylcarbonats ist.

**22.** Ein photochromer Gegenstand gemäß Anspruch 19, 20 oder 21, bei dem der Anteil der Heteropyran-Verbindung von 0.01 bis 0.5 Gew.-% beträgt, bezogen auf das Gewicht des polymeren Trägermaterials.

**23.** Ein photochromer Gegenstand gemäß einem oder mehreren der Ansprüche 19 bis 22, der eine weitere photochrome Verbindung, ausgewählt aus den Spiro(indolino)naphthoxazinen, Spiro(indolino)pyridobenzoxazinen, Spiro(indolino)benzoxazinen und Naphthopyranen, enthält.

**24.** Ein photochromer Gegenstand gemäß Anspruch 23, bei dem die zusätzliche photochrome Verbindung zu einem Anteil von 0.001 bis 0.5 Gew.-% vorliegt, bezogen auf das Gewicht des polymeren Trägermaterials.

**25.** Ein photochromer Gegenstand gemäß einem oder mehreren der Ansprüche 19 bis 24, der in Form einer Linse vorliegt.

**26.** Ein photochromer Gegenstand gemäß Anspruch 25, wobei die Linse eine Kontaktlinse ist.

## Revendications

**1.** Composé hétérobenzopyrannique répondant à la formule générale (I)

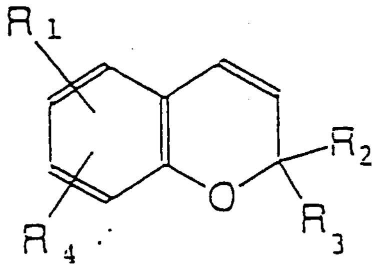

(I)

dans laquelle $R_1$ représente un groupe hétérocyclique à cinq chaînons contenant un ou plusieurs hétéroatomes et condensé avec la face f ou la face g du fragment benzo du squelette benzopyrannique, ledit groupe hétérocyclique étant condensé d'une manière telle que l'hétéroatome dans le groupe hétérocyclique est lié directement à un atome de carbone sur ledit fragment benzo, et le cas échéant, ledit groupe hétérocyclique est condensé en outre sur celui-ci avec un autre groupe carbocyclique ou hétérocyclique ou est substitué par un groupe de formule $R_4$ tel que défini ci-après ;
chacun des substituants $R_2$ et $R_3$ est choisi parmi un groupe phényle, un groupe 4-trifluorométhylphényle, un groupe 4-alkoxyphényle, un groupe 2,4-di(alkoxy)phényle ou un groupe 4-dialkylaminophényle, avec la condition qu'au moins un des substituants $R_2$ et $R_3$ soit choisi parmi un groupe 4-alkoxyphényle ou un groupe 2,4-di(alkoxy)phényle ; et
$R_4$ représente un atome d'hydrogène ou un groupe alkyle.

**2.** Composé hétérobenzopyrannique selon la revendication 1, dans lequel les substituants $R_2$ et $R_3$ sont choisis parmi un groupe 4-méthoxyphényle, un groupe 2,4-diméthoxyphényle et un groupe 4-diméthylaminophényle, avec la condition qu'au moins un des substituants $R_2$ et $R_3$ soit choisi parmi un groupe 4-méthoxyphényle ou un groupe 2,4-diméthoxyphényle.

**3.** Composé hétérobenzopyrannique selon la revendication 1 ou la revendication 2, dans lequel le noyau hétérocyclique à cinq chaînons dans le groupe $R_1$ est un noyau furannique, un noyau pyrrolique ou un noyau thiophénique.

**4.** Composé hétérobenzopyrannique selon la revendication 3, dans lequel l'hétéroatome du noyau furannique, pyrrolique ou thiophénique est attaché en position 7 du squelette benzopyrannique.

**5.** Composé hétérobenzopyrannique selon l'une quelconque des revendications précédentes, dans lequel le noyau hétérocyclique dans le groupe $R_1$ a un noyau benzénique condensé avec celui-ci ou un noyau cyclohexanique condensé avec celui-ci ou porte un ou plusieurs substituants alkyle.

**6.** Composé hétérobenzopyrannique selon l'une quelconque des revendications 3 à 5, dans lequel l'atome d'azote cyclique du noyau pyrrolique est substitué par un groupe alkyle.

**7.** Composé hétérobenzopyrannique selon la revendication 6, dans lequel le groupe alkyle est un groupe méthyle.

**8.** Composé hétérobenzopyrannique selon l'une quelconque des revendications précédentes, dans lequel $R_4$ est un atome d'hydrogène ou un groupe méthyle.

**9.** 3,3-dianisyl-3H-[chroméno[6,5-b]benzofuranne].

**10.** 3,3-dianisyl-3H-[chroméno[6,7-b]benzofuranne].

**11.** 3-anisyl-3-(2,4-diméthoxyphényl)-3H-[chroméno[6,5-b]benzofuranne].

**12.** 3-anisyl-3-(2,4-diméthoxyphényl)-3H-[chroméno[6,7-b]benzofuranne].

**13.** 3,3-dianisyl-6-méthyl-3H-[pyranno[2,3-b]-9H-5,6,7,8-tétrahydrocarbazole].

**14.** 3,3-dianisyl-10-méthyl-3H-[pyranno[3,2-b]-9H-5,6,7,8-tétrahydrocarbazole].

**15.** 3,3-dianisyl-7H-[pyrrolo[2,3-f]chromène].

**16.** 3-anisyl-3-(2,4-diméthoxyphényl)-6-méthyl-3H-[chroméno[6,7-b]benzofuranne].

**17.** 3-anisyl-3-(2,4-diméthoxyphényl)-8,9-diméthyl-3H-[pyranno[3,2-e]benzothiophène].

**18.** Procédé de préparation d'un composé hétérobenzoyrannique de formule (I) tel que défini dans la revendication 1, ledit procédé comprenant la condensation d'un composé hydroxy de formule générale (III)

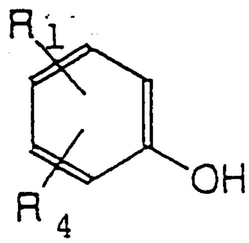

(III)

dans laquelle $R_1$ et $R_4$ sont tels que définis dans la revendication 1, l'hétéroatome dans le noyau hétérocyclique du groupe $R_1$ étant situé en position para ou méta par rapport au groupe hydroxy sur le noyau benzénique, avec un alcool propargylique de formule générale (IV)

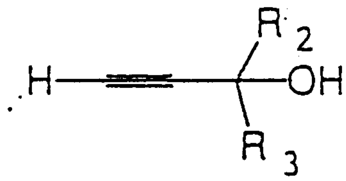

(IV)

dans laquelle $R_2$ et $R_3$ sont tels que définis dans la revendication 1, en présence d'alumine acide, d'acide trifluoroacétique ou d'un autre catalyseur acide analogue.

**19.** Article photochromique comprenant un matériau hôte polymère ayant un composé hétéropyrannique selon l'une quelconque des revendications 1 à 17 incorporé dans celui-ci ou appliqué sur celui-ci.

**20.** Article photochromique selon la revendication 19, dans lequel le matériau hôte polymère est choisi parmi des polymères de monomères allylcarbonate de polyol, polyacrylates, poly(acrylates d'alkyle), acétate de cellulose, triacétate de cellulose, acétopropionate de cellulose, acétobutyrate de cellulose, poly(acétate de vinyle), poly(alcool vinylique), polyuréthannes, polycarbonates, téréphtalate de polyéthylène, polystyrène, copolymères de styrène/méthacrylate de méthyle, copolymères de styrène/acrylonitrile et polyvinylbutyral.

**21.** Article photochromique selon la revendication 20, dans lequel le matériau hôte polymère est un polyuréthanne ou un polymère de bis(allylcarbonate) de diéthylèneglycol.

**22.** Article photochromique selon la revendication 19, 20 ou 21, dans lequel la quantité de composé hétérobenzopyrannique est de 0,01 à 0,5 % en poids par rapport au poids du matériau hôte polymère.

**23.** Article photochromique selon l'une quelconque des revendications 19 à 22, comprenant un composé photochromique supplémentaire choisi parmi des spiro(indoline)naphtoxazines, spiro(indolino)pyridobenzoxazines, spiro(indolino)benzoxazines et naphtopyrannes.

**24.** Article photochromique selon la revendication 23, dans lequel le composé photochromique supplémentaire est présent en une quantité de 0,001 à 0,5 % en poids par rapport au poids du matériau hôte polymère.

**25.** Article photochromique selon l'une quelconque des revendications 19 à 24, qui se présente sous la forme d'une lentille.

**26.** Article photochromique selon la revendication 25, la lentille étant une lentille ophtalmique.